# EUROPÄISCHE PATENTANMELDUNG

(11) **EP 2 107 065 A1**
(43) Veröffentlichungstag der Anmeldung: **07.10.2009**
(21) Anmeldenummer: 08006674.9
(22) Anmeldetag: 01.04.2008
(51) Int. Cl.: C07F 15/00

(54) **Ruthenium-Komplexe mit (P-P)-koordinierten Di-Phosphor-Donorliganden sowie Verfahren zu ihrer Herstellung**

(71) Anmelder: Umicore AG & Co. KG, 63457 Hanau-Wolfgang (DE)
(72) Erfinder: Rivas-Nass, Andreas, 69198 Schriesheim (DE); Karch, Ralf, 63801 Kleinostheim (DE); Winde, Roland, 60599 Frankfurt (DE); Doppiu, Angelino, 63517 Rodenbach (DE); Schneider, Tina, 63505 Langenselbold (DE)
(74) Vertreter: Starz, Karl Anton

(57) **Zusammenfassung**

Die Erfindung betrifft Rutheniumkomplexe mit einem chiralen Di-Phosphor-Donorliganden wobei das Ruthenium die Oxydationsstufe (+II) besitzt und der chirale Di-Phosphor-Donorligand eine bidentate P-P-Koordination am Ruthenium besitzt. Die Rutheniumkomplexe liegen in zwei Formen vor (kationischer Typ A und neutraler Typ B), sind zyklisch und weisen mit dem Di-Phosphor-Donorliganden einen vier- bis sechsgliedrigen Ring auf. Die chiralen Di-Phosphor-Donorliganden sind ausgewählt aus der Gruppe der Diphosphine, Diphospholane, Diphosphite, Diphosphonite und Diazaphospholane. Weiterhin werden Herstellungsverfahren für die Rutheniumkomplexe des Typs A und B beschrieben, die auf Ligandenaustauschreaktionen beruhen.

Die Ru-Komplexe finden Verwendung als Katalysatoren für die homogene asymmetrische Katalyse zur Herstellung von organischen Verbindungen.

## Beschreibung

Die vorliegende Erfindung betrifft Rutheniumkomplexe für die homogene Katalyse, insbesondere P-P-koordinierte ringförmige Rutheniumkomplexe mit chiralen Di-Phosphor-Donorliganden. Ein weiterer Gegenstand der Erfindung sind Verfahren zu ihrer Herstellung sowie die Verwendung dieser Komplexe als Katalysatoren für die homogene Katalyse, insbesondere für die enantioselektive Hydrierung.

Chirale Di-Phosphor-Donorliganden haben sich als wertvolle Liganden für katalytisch wirksame Metallkomplexe erwiesen, die in der homogenen Katalyse zur enantioselektiven Hydrierung von organischen Verbindungen verwendet werden. Einsatzbereiche sind die Herstellung von Zwischenprodukten oder Wirkstoffen, beispielsweise Pharmazeutika, Pestizide, Aromen oder Riechstoffe.

Bei der enantioselektiven Hydrierung werden diese Di-Phosphor-DonorLiganden zusammen mit geeigneten Edelmetallkomplexen eingesetzt.

Als metallorganische Ru-Verbindungen werden bei diesen Hydrierungen z.B. [Ru(COD)Y₂]ₓ, [Ru(NBD)Y₂]ₓ, [Ru(Aromat)Y₂]ₓ, oder [Ru(COD)(2-Methylallyl)₂] (mit X = 2; Y = Halogenid, COD = 1,5-Cyclooctadien, NBD = Norbornadien, Aromat = z.B. p-Cumol oder ein anderes Benzolderivat) eingesetzt.

Aus der EP 1622920B1 sind Übergangsmetallkomplexe mit Ferrocenyl-diphosphinliganden bekannt. Komplexe mit einer speziellen P-P-Koordination der Phosphinliganden werden nicht beschrieben.

Aus der Literatur sind Ru-Komplexe mit (P-P)-koordinierten Di-Phosphor-Donorliganden bekannt.

M. Sato und M. Asai (J. of Organometallic Chemistry 508 (1996) S. 121-127) beschreiben Permethylcyclopentadienyl-Ru(II)-Komplexe mit dppf-, BINAP- und DIOP-Liganden. Bedingt durch den Permethyl-Cyclopentadienyl-Rest sind diese Komplexe sehr stabil, ein Einsatz für die katalytische Hydrierung wird nicht beschrieben.

C. Standfest-Hauser, C. Slugovc et al. (J. Chem. Soc. Dalton Trans., 2001, S. 2989-2995) berichten über ringförmige Ru(II)-Halbsandwich-Komplexe, die ebenfalls einen Cyclopentadienyl-Liganden sowie chelatbildende Phosphinoamin-Liganden aufweisen. Diese Komplexe enthalten einen Cyclopentadienylliganden ("Cp-Liganden") und sind für den Einsatz als Katalysatoren zur homogenen asymmetrischen Hydrierung wenig geeignet. Sie werden normalerweise in einer speziellen Katalysereaktion (der sog. Transfer-Hydrierung) eingesetzt, wobei der Cp-Ligand den Katalysator stabilisiert und während der Katalyse am Metall koordiniert sein muss.

J. B. Hoke et al. (J. of Organomet. Chem., 1993, 455, S. 193-196) beschreiben Ruthenium-Komplexe, bei denen BINAP-Liganden mit dem Ru einen siebengliedrigen Ring ausbilden. Die Komplexe enthalten eine Cyclopentadienyl-Gruppe. Sie sind sehr stabil, aber in der katalytischen Hydrierung wenig aktiv und teilweise unselektiv, da der Cp-Ligand sehr stark am Metal gebunden ist.

P. S. Pregosin et al (Organometallics, 1997, 16, S. 537-543) berichtete über einen Ruthenium-Komplex mit dem MeO-BIPHEP Liganden, der zusätzlich einen Cyclooctadienyl-Rest aufweist. Der Komplex ist nur mit einem aufwendigen Prozess herstellbar und sehr luft- und feuchtigkeitsempfindlich. Ein Einsatz für die katalytische Hydrierung wird nicht beschrieben. Ein ähnlicher Komplex mit dem BINAP-Ligand wurde von S. H. Bergens et al. beschrieben (Organometallics, 2004, 23, S. 4564-4568). Bei der Synthese wurde ein Acetonitril-Komplex isoliert, der in der Katalyse wenig aktiv ist.

A. Salzer et al. (Organometallics, 2000, 19, S. 5471-5476) berichtete über die Herstellung eines siebengliedrigen BINAP-Ruthenium-Komplexes aus einer Ru-(Bis-Pentadienyl)-Verbindung als Ausgangskomplex.

D. A. Dobbs et al. (Angew. Chem. 2000, Band 112, S. 2080-2083) beschreiben einen rutheniumhaltigen Präkatalysator mit einem P-P-koordinierten DuPhos-Liganden. Die ringförmige Verbindung stellt eine Ru-Hydridspezies dar und weist einen ungeladenen Cycloctatrienylliganden auf. Sie ist sehr empfindlich und wurde in einer Handschuhbox (Inertgas Ar mit < 1 ppm Sauerstoff) hergestellt. Solche empfindlichen Verbindungen sind für den industriellen Einsatz wenig geeignet.

In der WO 00/37478 werden zwar Übergangsmetallkomplexe mit einem Metallatom der 7. oder 8. Nebengruppe beschrieben, bei denen beide P-Atome Diphosphinliganden gleichzeitig an das Zentralatom koordiniert sind; die Komplexe werden jedoch weder isoliert noch charakterisiert. Es wird kein Herstellverfahren angegeben, vielmehr werden die Komplexe kurz vor ihrem Einsatz durch Zusammengeben der Liganden und der entsprechenden Übergangsmetallsalze im Reaktionslösungsmittel erzeugt ("*in-situ*"). Diese *in*-*situ*-Verfahren sind Stand der Technik. Es sind bisher wenige Untersuchungen zur Struktur der *in*-*situ* erzeugten Metallkomplexe durchgeführt worden; die entsprechenden Komplexe wurden nicht isoliert, sondern im Reaktionsgemisch direkt zur homogene Katalyse, insbesondere zur katalytischen Hydrierung verwendet. Die mechanistischen Studien werden mit Modell-Systemen durchgeführt, die nicht der realen aktive katalytischen Spezies entsprechen.

Nachteile der bisher beschriebenen katalytischen Hydrierverfahren und der hierbei eingesetzten Katalysatoren sind insbesondere die geringe Reaktivität, die geringen Enantioselektivitäten sowie ein hoher Verbrauch an edelmetallhaltigem Katalysator, d.h. ein niedriges "substrate/catalyst" (S/C)-Verhältnis. Weiterhin werden lange Hydrierzeiten benötigt. Zudem sind viele der beschriebenen Komplexe synthetisch schwierig herzustellen, luftempfindlichen und für den industriellen Einsatz wenig geeignet.

Es war daher eine Aufgabe der vorliegenden Erfindung, verbesserte Katalysatoren für die homogene, asymmetrische Hydrierung bereitzustellen, die die oben genannten Nachteile überwinden. Eine weitere Aufgabe war die Bereitstellung von geeigneten Herstellverfahren für die erfindungsgemäßen Katalysatoren. Insbesondere sollten diese Verfahren industriell einsetzbar, umweltfreundlich und kostengünstig sein.

Diese Aufgabe wird durch die Bereitstellung der Rutheniumkomplexe des Typs A und B gemäß Anspruch 1 der Erfindung gelöst. Weiterhin werden Verfahren zur Herstellung der erfindungsgemäßen Komplexe des Typs A und B in den Ansprüchen 12 und 22 bereitgestellt. Bevorzugte Ausführungsformen der Komplexe sowie der Verfahren sind in den jeweiligen Unteransprüchen beschrieben.

Die vorliegende Erfindung beschreibt Ruthenium-Komplexe mit einem chiralen Di-Phosphor-Donorliganden des Typs P(1)-P(2) für die homogene Katalyse. Die Komplexe liegen in zwei verschiedenen Typen vor (Typ A und Typ B), wobei das Ruthenium die Oxidationsstufe +II besitzt und die Di-Phosphor-Donorliganden eine bidentate P-P-Koordination am Ru zeigen. Die erfindungsgemäßen Rutheniumkomplexe sind zyklisch und weisen zusammen mit den Di-Phosphor-Donorliganden einen vier- bis sechsgliedrigen Ring auf.

Bei der Suche nach wohldefinierten Ru-Komplexen mit diesen chiralen Di-Phosphor-Donorliganden hat sich überraschenderweise gezeigt, dass unter bestimmten Bedingungen eine gleichzeitige Koordination beider P-Atome der Di-Phosphor-Donorliganden an ein Ru-Zentralatom erreicht werden kann. Dabei erhält man ringförmige Ru-Komplexe, die einen vier- bis sechsgliedrigen Ring in ihrer Struktur aufweisen und die eine sehr gute katalytische Aktivität bei der homogenen Katalyse zeigen.

Den Effekt der P-P-Koordination erhält man durch den Einsatz von Di-Phosphor-Donorliganden P(1)-P(2), die sterisch in der Lage sind, mit dem Ru-Zentralatom einen vier- bis sechsgliedrigen Ring zu bilden. Im Rahmen dieser Anmeldung weisen dabei auch Ru-Komplexe mit Ferrocenyl-Phosphin-Liganden, die Phosphingruppen an den unterschiedlichen Cp-Ringen besitzen, einen sechsgliedrigen Ring auf. Die Ferrocen-Einheit P-C-Fe-C-P stellt dabei fünf Ringatome des sich ausbildenden Zyklus zur Verfügung. Die hierbei verwendete Zählweise der Ringglieder ist in Abb. 1 schematisch dargestellt.

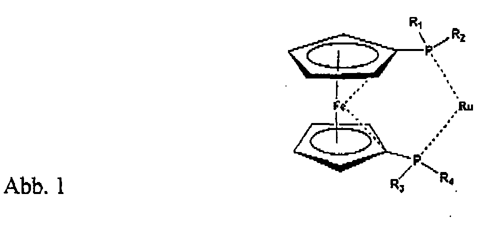

Der chirale Di-Phosphor-Donorligand P(1)-P(2) ist allgemein ausgewählt aus der Gruppe der Diphosphine, Diphospholane, Diphosphite, Diphosphonite und Diazaphospholane.

Beispiele für geeignete Di-Phosphor-Donorliganden zur Herstellung von viergliedrigen ringförmigen Ru-Komplexen sind die Liganden der Familien MiniPhos und Trichickenfootphos.

Beispiele für geeignete Di-Phosphor-Donorliganden zur Herstellung von fünfgliedrigen ringförmigen Ru-Komplexen sind die Liganden der Familien DIPAMP, Norphos, PROPHOS, DuPHOS, Chiraphos, Bis-P*-family, DepyPhos (oder DeguPhos), RoPhos, CATAXIUM, ButiPhane, (1,2-ethylen)-BinaPhane, (1,2-phenylen)-BinaPhane, Binapine, TangPhos, BPE, BasPhos, MalPhos, Me-KetalPhos, Helmchen-Liganden, PennPhos, UCAP, Hoge Liganden, sowie CNR-Phos. Der Einsatzbereich der Erfindung ist jedoch nicht auf diese Liganden beschränkt.

Beispiele für geeignete Di-Phosphor-Donorliganden zur Herstellung von sechsgliedrigen ringförmigen Ru-Komplexen sind die Liganden der Familien BDPP (oder SKEWPHOS), BPPFOH, MandyPhos, JosiPhos, FerroTANE Me-f-KetalPhos, Ferrocelane, Trifer sowie (1, 1 -ferrocene)-BinaPhane. Der Einsatzbereich der Erfindung ist jedoch nicht auf diese Liganden beschränkt.

Eine bevorzugte Ausführungsform der Erfindung sind Rutheniumkomplexe, die fünf- oder sechsgliedrige Ringe aufweisen. Bevorzugte Ligandensysteme sind die Liganden der DepyPhos (Deguphos)-Familie, die fünfgliedrige Ringe bilden, sowie die Josiphos- und Mandyphos-Liganden, die zu sechsgliedrigen Ringen führen.

Die oben angegebenen Di-Phosphor-Donorliganden können axiale, zentrale und/oder planare Chiralität aufweisen und sind in den meisten Fällen kommerziell erhältlich. Beim Einsatz dieser Liganden im erfindungsgemäßen Herstellverfahren erhält man zyklische Ru-Komplexe, die einen vier- bis sechsgliedrigen Ring in ihrer Struktur besitzen. Jedoch sind auch andere chirale Di-Phosphor-Donorliganden geeignet, sofern sie die Bildung eines vier- bis sechsgliedrigen Ringes ermöglichen.

Weiterhin umfasst die vorliegende Erfindung ein Verfahren zur Herstellung der erfindungsgemäßen Rutheniumkomplexe des Typs A und B, wobei spezielle Ru-Ausgangsverbindungen mit einem chiralen Di-Phosphor-Donorliganden umgesetzt werden bzw. der Rutheniumkomplex des Typs A mit einem weiteren Ligand umgesetzt wird.

Den Effekt der P-P-Koordination in den erfindungsgemäßen Rutheniumkomplexe erreicht man beim vorliegenden Herstellverfahren durch den Einsatz einer speziellen Ru-Ausgangsverbindung, in der das Ru-Zentralatom in der Oxidationsstufe +II vorliegt und die mindestens drei neutrale Liganden L_{D} aufweist. Sie besitzt die folgende allgemeine Formel

[Z-Ru-(L_{D})ₙ]⁺E⁻

wobei
Ru in der Oxydationsstufe +II vorliegt,
n eine ganze Zahl mit n ≥ 3 darstellt,
L_{D} ein neutraler Ligand,
Z ein π-gebundener anionischer offener Pentadienyl-Ligand und
E⁻ das Anion einer Sauerstoffsäure oder Komplexsäure bedeutet.

Die mindestens drei neutralen Liganden L_{D} gehören zur Klasse der 2-Elektronen-Donor-Liganden, wie beispielsweise sekundäre oder tertiäre Phosphine (Liganden des Typs PR₂H oder PR₃ wie beispielsweise Triphenylphosphin) oder N-heterocyclische Carbenliganden (sog. NHC-Liganden). Vorzugsweise sind L_{D} Liganden, die an das Ru-Zentralatom schwach gebunden sind. Mindestens zwei dieser Liganden werden bei der Umsetzung mit einem chiralen Di-Phosphor-Donorliganden substituiert. Beispiele für geeignete Liganden L_{D} sind Liganden aus der Gruppe der Nitrile, Isonitrile, Alkohole, Ether, Amine, Säureamide, Lactame und Sulfone; beispielsweise Acetonitril (CH₃CN), Diethylether (DEE), Wasser (H₂O), oder Aceton. Es können auch zyklische Liganden wie beispielsweise Tetrahydrofuran (THF), Dioxan, Pyridin, Imidazol oder Thiophen eingesetzt werden. Es sind auch gemischte Systeme mit unterschiedlichen Ligandentypen möglich.

Weiterhin weist die Ru-Ausgangsverbindung einen π-gebundenen anionischen (d.h. einfach negativ geladenen) Liganden Z auf. Z umfasst offene (d.h. nicht zyklische) Pentadienyl-Liganden. Solche Liganden können substituiert oder unsubstituiert sein. Sie weisen ein delokalisiertes π-Elektronensystem auf. Die substituierten Pentadienyl-Liganden können dabei mono-, di- oder trisubstituiert sein.

Bevorzugte substituierte Pentadienylliganden Z sind 1-Methyl-pentadienyl, 2-Methyl-pentadienyl, 3-Phenyl-pentadienyl, 2,4-Dimethyl-pentadienyl- oder 2,3,4-Trimethyl-pentadienyl.

Geschlossene, ringförmige aromatische π-Systeme wie beispielsweise substituierte oder unsubstituierte Cyclopentadienylliganden sind als Liganden Z nicht geeignet. Es hat sich gezeigt, dass solche Cyclopentadienylliganden aufgrund der hohen Elektronendichte des vorhandenen aromatischen π-Elektronensystems sehr stark an das Zentralatom Ru gebunden ist. Damit wird der Zugang von Reaktanden während der Katalysereaktion, beispielsweise bei einer katalytischen Hydrierung, behindert. So wird beispielsweise in einer Hydrierungsreaktion die Koordination der zu hydrierenden Verbindung an das Ru-Atom erschwert, wodurch der entsprechende Ru-Komplex eine geringe Reaktivität als Katalysator aufweist.

Die für das erfindungsgemäße Verfahren verwendete Ru-Ausgangsverbindung liegt als Kation mit einfach positiver Ladung vor und besitzt als weiteren Bestandteil E⁻ (das Anion einer Sauerstoffsäure oder Komplexsäure) als Gegenion. Beispiele für E⁻ sind HSO₄⁻, CF₃SO₃⁻, ClO₄⁻, CF₃COO⁻, CH₃COO⁻, BF₄⁻, B(Aryl)₄⁻, SbF₆⁻ oder PF₆⁻.

Beispiele für geeignete Ru-Ausgangsverbindungen sind [Ru(2,4-Dimethyl-pentadienyl) (CH₃CN)₃]⁺BF₄⁻, [Ru(2,4-Dimethyl-pentadienyl) (H₂O)₃]⁺BF₄⁻ oder [Ru (2,4-Dimethyl-pentadienyl)(Aceton)₃]⁺BF₄⁻. Die Ru-Ausgangsverbindung kann nach literaturbekannten Verfahrensschritten zunächst aus bekannten, kommerziell erhältlichen Ru-Precursor-Verbindungen (z.B. Bis(η5-(2,4-Dimethyl-pentadienyl)-Ru) durch Umsetzung mit dem betreffenden Liganden L_{D} hergestellt werden.

Es wurde gefunden, dass bei der Reaktion der oben beschriebenen Ru-Ausgangsverbindung mit den geeigneten chiralen Di-Phosphor-Donorliganden (nachfolgend abkürzend mit P(1)-P(2) dargestellt) die erfindungsgemäßen Ru-Komplexe erhalten werden. Die Bildung des Komplexes des Typs A erfolgt durch Ligandenaustausch im Herstellverfahren gemäß Gl. (1):

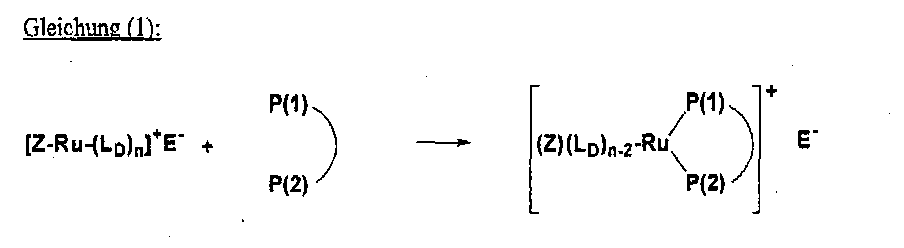

Gemäß Gl. (1) erhält man den erfindungsgemäßen Ru-Komplex des Typs A. Dieser Komplex ist kationisch, d.h. einfach positiv geladen. Bei der Herstellung setzt man den chiralen Di-Phosphor-Donorliganden mit der bereits beschriebenen Ru-Ausgangsverbindung um, wobei mindestens zwei der Liganden L_{D} bei der Umsetzung substituiert werden. Besitzt die Ru-Ausgangsverbindung mehr als zwei Liganden L_{D}, so bleiben die restlichen Liganden L_{D} an das Ruthenium koordiniert. Sie können in einem nachfolgenden Schritt ausgetauscht bzw. eliminiert werden. Dabei erhält man den erfindungsgemäßen Ru-Komplex des Typs B.

Die Herstellung der Ru-Komplexe des Typs A und B erfolgt vorzugsweise unter Schutzgasatmosphäre unter Verwendung der Schlenk-Technik mit sauerstofffreien Lösungsmitteln. Dies ist jedoch nicht in jedem Falle notwendig.

Typischerweise werden zur Herstellung des Typs A die Di-Phosphor-Donorliganden mit der Ru-Ausgangsverbindung in einem geeigneten Lösungsmittel bei Temperaturen im Bereich von -80°C bis 80°C, vorzugsweise im Bereich von 20 bis 60°C und besonders bevorzugt bei Raumtemperatur (25°C) unter Rühren umgesetzt.

Geeignete Lösungsmittel sind chlorierte Kohlenwasserstoffe wie Chloroform, Methylenchlorid, Trichlorethan oder Dichlorethan. Vorzugsweise werden jedoch dipolare, aprotische Lösungsmittel wie Aceton, THF oder Dioxan verwendet. Die Reaktionszeiten liegen im Bereich von 1 Stunde bis 48 Stunden, vorzugsweise im Bereich von 1 Stunde bis 24 Stunden. Es kann vorteilhaft sein, bei der Herstellung der Ru-Komplexe des Typs A den Liganden etwas im Überschuss einzusetzen, wobei der Überschuss zwischen 1 bis 10 Mol.-% (bezogen auf die Ru-Ausgangsverbindung) betragen kann. Die nachfolgenden Isolations-, Wasch- und Reinigungsschritte sind dem Fachmann geläufig. Zur Entfernung der Lösungsmittelreste trocknet man im Vakuum.

Weiterhin umfasst die vorliegende Erfindung Ru-Komplexe des Typs B. Diese Komplexe sind insgesamt elektroneutral und weisen neben dem P-P-koordinierten chiralen Di-Phosphor-Donorliganden, dem π-gebundenen anionischen Pentadienyl-Liganden Z und ggf. dem Liganden L_{D} noch einen negativ geladenen Liganden L_{Z} auf. Dieser Ligand L_{Z} wird durch Substitution eines der Liganden L_{D} mit einem Anion, vorzugsweise mit einem Halogenidion (Fluorid, Chlorid, Bromid oder Jodid) oder einem Pseudohalogenidion (z.B. CN⁻, SCN⁻, Cyanat, Isocyanat etc) eingeführt.

Vorzugsweise wird der Ru-Komplex des Typs B durch einen Ligandenaustausch aus dem Komplex des Typs A hergestellt, beispielsweise durch den Austausch eines Acetonitril-Moleküls durch Jodid (vgl. hierzu Beispiel 2). Die Gleichung (2) beschreibt den Austausch des Liganden L_{D} durch einen negativ geladenen Liganden L_{Z}):

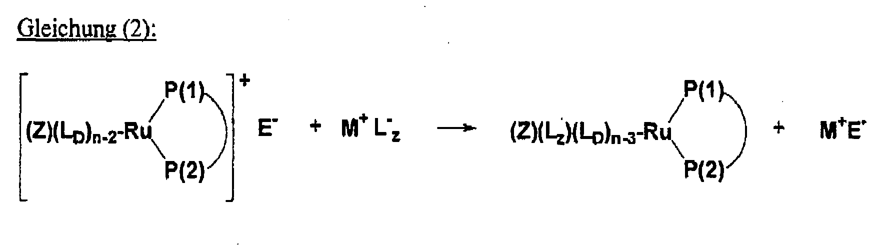

Zur Durchführung der Reaktion nach Gleichung (2) wird der kationische Ru-Komplex (Typ A) in geeigneten Lösungsmitteln wie chlorierten Kohlenwasserstoffen (z.B. Chloroform, Methylenchlorid, Trichlorethan oder Dichlorethan), Alkoholen, Ketonen (z.B. Aceton) oder zyklischen Ethern (z.B. THF oder Dioxan) umgesetzt. Vorzugsweise werden jedoch wässrige Lösungsmittelgemische, insbesondere ein Gemisch eines dipolaren, aprotischen Lösungsmittels mit vollentsalztem Wasser verwendet. Dabei wird der kationische Ru-Komplex (Typ A) beispielsweise in Aceton/Wasser (2:1) gelöst und mit dem Liganden L_{Z} bei Temperaturen im Bereich von 0° bis 50°C umgesetzt. Der Ligand L_{z} wird vorzugsweise in Form eines Salzes M⁺L_{z} zugesetzt, beispielsweise in Form eines Alkali- oder Ammoniumhalogenides. Das Produkt fällt in der Regel aus und kann abgetrennt werden.

Die wässrigen Lösungsmittelgemische sind aus Umweltschutz- und Arbeitsschutzgründen für industrielle Syntheseverfahren besonders geeignet. Es hat sich überraschenderweise gezeigt, dass die bisher verwendeten chlorierte Kohlenwasserstoffe ohne Ausbeuteverluste al Lösungsmittel ersetzt werden können.

Die erfindungsgemäßen Ru-Komplexe des Typs A 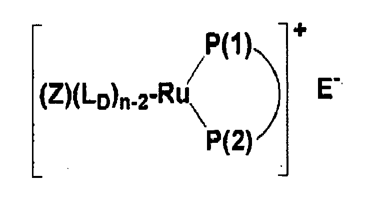 und des Typs B 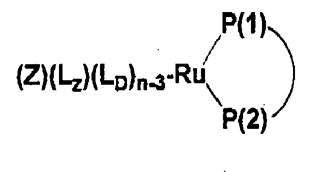 wobei jeweils
Ru in der Oxydationsstufe +II vorliegt,
P(1)-P(2) ein chiraler Di-Phosphor-Donorligand,
n eine ganze Zahl mit n ≥ 3,
L_{D} mindestens ein neutraler Ligand,
Z ein π-gebundener anionischer offener Pentadienyl-Ligand
E⁻ ein Anion einer Sauerstoffsäure oder Komplexsäure und
L_{Z} mindestens ein anionischer Ligand ist,
und der chirale Di-Phosphor-Donorligand P(1)-P(2) eine bidentate P-P-
Koordination aufweist und mit dem Ruthenium einen vier- bis sechsgliedrigen
Ring bildet,
stellen wirksame homogene Katalysatoren für die asymmetrische Hydrierung von prochiralen organischen Verbindungen dar.

Ein gemeinsames Kennzeichen der erfindungsgemäßen Ru-Komplexe des Typs A und B ist das Vorhandensein des anionischen offenenen Pentadienyl-Ligand im Komplex. Es hat sich überraschenderweise gezeigt, dass dadurch eine höhere Reaktivität der Ru-Komplexe in der Katalyse, insbesondere bei der asymmetrischen Hydrierung, einhergeht. Vermutlich ist der offene Pentadienylligand, im Gegensatz zu geschlossenen Ligandensystemen wie Cyclopentadienyl und Cyclooctadienyl, einfacher zu destabilisieren, sodass er eine Koordinationsstelle für das zu hydrierenden Substratmolekül bereitstellen kann. Überraschendweise hat es sich gezeigt, dass auch die Liganden L_{D} und/oder L_{Z} eine sehr wichtige Rolle spielen. Vermutlich wird eine Koordinationsstelle damit blockiert, sodass das Substrat nur in einer bestimmten Weise am Metal koordinieren kann, wodurch die Enantioselektivität erhöht wird.

Die erfindungsgemäßen Ru-Komplexe des Typs A und B finden daher Verwendung als Katalysatoren für die homogene asymmetrische Katalyse, beispielsweise zur enantioselektiven Hydrierung von Mehrfachbindungen. Unter Mehrfachbindungen werden in der vorliegenden Erfindung Doppelbindungen zwischen einem Kohlenstoffatom und einem weiteren Kohlenstoffatom (C=C) oder Sauerstoffatom (C=O) oder Stickstoffatom (C=N) verstanden.

Weiterhin können die erfindungsgemäßen Rutheniumkomplexe auch als Katalysatoren für andere asymmetrische Reaktionen verwendet werden. Hierzu gehören C-C, C-O, C-N, C-P, C-Si, C-B oder C-Halogen Bindungsknüpfungsreaktionen. Beispiele sind asymmetrische Cyclisierungsreaktionen, asymmetrische Oligomerisierungen und asymmetrische Polymerisationsreaktionen.

Die erfindungsgemäßen Ruthenium(II)-Komplexe des Typs A und B werden als definierte Verbindungen verwendet. Im Vergleich dazu zeigen Ru-Komplexe, die zusammen mit den Di-Phosphor-Donorliganden im sog. *in-situ*-Verfahren verwendet werden, schlechtere katalytische Eigenschaften.

Die nachfolgenden Beispiele sollen die Erfindung erläutern, ohne den Schutzbereich einzuschränken.

### BEISPIELE

### Beispiel 1:

### Herstellung von (η5-2,4-Dimethylpentadienyl)-(CH₃CN)-(Josiphos-SL-J212-1)-ruthenium(II)-tetrafluoroborat

### a) Herstellung von (η4 -2,4-Dimethylpentadien-η2-C, H)-(η5-2,4-dimethyl-pentadienyl)ruthenium-tetrafluoroborat

In einem 100 ml Schlenkrohr mit Magnetrührer werden 1,1 g (3,77 mmol) Bis-(η5-2,4 dimethylpentadienyl) ruthenium (UMICORE, Hanau) in 50 ml Diethylether gelöst. Bei Raumtemperatur tropft man innerhalb von 10 Minuten 0,51 ml (3,77 mmol) einer 54%-igen HBF₄ - Et₂O Lösung hinzu (Fa. Aldrich). Nach vollständiger Zugabe lässt man absitzen und prüft die Vollständigkeit der Fällung durch Zugabe eines weiteren Tropfens HBF₄ - Et₂O. Das überstehende Lösungsmittel wird abgezogen und der Feststoff zweimal mit Diethylether gewaschen. Den hellgelben Rückstand trocknet man im Vakuum. Ausbeute 1,43 g (100%).

### b) Herstellung des Acetonitril-Komplexes (η5 -2,4-Dimethylpentadienyl)-(CH₃CN)₃ ruthenium(II) tetrafluoroborat (Ausgangsverbindung)

0,41 g (1,1 mmol) (η4 -2,4-Dimethylpentadien-η2-C, H)-(η5-2,4-dimethylpentadienyl) ruthenium-tetrafluoroborat, hergestellt in Schritt a), werden mit 10 ml Acetonitril versetzt. Man rührt die orangefarbene Lösung 10 Minuten, entfernt das Lösungsmittel im Vakuum und erhält einen orangefarbenen Feststoff. Ausbeute 0,44 g (100%).

### c) Herstellung von (η5-2,4-Dimethylpentadienyl)-(CH₃CN)-(Josiphos-SL-J212-1)-ruthenium(II)-tetrafluoroborat

In einem 50 ml Rundkolben mit Magnetrührer wird der Acetonitril-Komplex (η5 -2,4-Dimethylpentadienyl)-(CH₃CN)₃ ruthenium(II)-tetrafluoroborat in 15 ml Methylenchlorid gelöst und mit 200 mg (0,38 mmol) Josiphos SL-J212-1 (Fa. Solvias, Basel, CH) bei Raumtemperatur für 6 Stunden gerührt. Zu der Lösung werden dann 50 ml n-Hexan getropft. Es fällt ein rot-oranger Feststoff aus, der filtriert wird. Das Restlösemittel wird im Vakuum bei Raumtemperatur entfernt. Man erhält ein Produkt als Mischung dreier Diastereomere, Ausbeute 95%. Die P-P-Koordination des Liganden (und damit das Vorliegen eines 6-gliedrigen Ringes) wird durch das ³¹P NMR belegt.

### Charakterisierung:

³¹P NMR (CD₂Cl₂) δ: 10.32 ppm (d, *J*_{PP}= 28.5 Hz), 11.80 ppm (d, *J*_{PP}= 31.0 Hz), 20.10 ppm (d, *J*_{PP}= 29.8 Hz), 75.79 ppm (d, *J*_{PP}= 31.0 Hz), 89.55 ppm (d, *J*_{PP}= 28.5 Hz), 94.75 ppm (d, *J*_{PP}= 29.8 Hz)

### d) Verwendung zur katalytischen Hydrierung

Der nach Beispiel 1 hergestellte (η5-2,4-Dimethylpentadienyl)-(CH₃CN)-(Josiphos-SL-J212-1)- ruthenium(II)-tetrafluoroborat-Komplex wird zur asymmetrischen Hydrierung von *E*-2-Methyl-2-butensäure verwendet. Die Hydrierung erfolgt im Autoklaven unter 50 bar Wasserstoff, Lösungsmittel Methanol, Temperatur 50°C. Nach 20 Stunden Reaktionszeit wird der Wasserstoffdruck entfernt. Der erfindungsgemäße Komplex liefert sehr gute Ergebnisse bei der Verwendung als Katalysator zur enantioselektiven katalytischen Hydrierung.

### Beispiel 2

### Herstellung von (η5-2,4-Dimethylpentadienyl)-(Iodo)-(Josiphos SL-J212-1)-ruthenium(II)

In einem 25 ml Rundkolben mit Magnetrührer wir der nach Beispiel 1b) hergestellte Acetonitril-Komplex (η5 -2,4-Dimethylpentadienyl)-(SL-J212-1)-(CH₃CN)-ruthenium (II) tetrafluoroborat (150 mg, 0.18 mmol) in 4 ml Aceton und 2 ml Wasser gelöst und mit Lithiumiodid (LiI, 28.5 mg, 0.21 mmol) bei Raumtemperatur für 3 Stunden gerührt. Die Lösung wird zur Trockne bei Raumtemperatur im Vakuum eingeengt. Der Rückstand wird dreimal mit 1 ml Wasser gewaschen. Man erhält das Produkt in Form einer diastereomerenreinen Verbindung. Ausbeute 90%. Die P-P-Koordination des SL-J-212-1 wird durch das ³¹P-NMR belegt.

### Charakterisierung:

³¹P NMR (CD₂Cl₂) δ: 7.51 ppm (d, *J*_{PP}= 35.9 Hz), 84.30 ppm (d, *J*_{PP}= 35.9 Hz),

### Verwendung zur katalytischen Hydrierung:

Der nach Beispiel 2 hergestellte (Josiphos SL-J-212-1) (Iodo) ruthenium(II)-Komplex wird zur asymmetrischen Hydrierung von *E*-2-Methyl-2-butensäure verwendet. Der Komplex liefert sehr gute Ergebnisse als Katalysator zur enantioselektiven katalytischen Hydrierung.

### Beispiel 3

### Herstellung von (η5-2,4-Dimethylpentadienyl)-(CH₃CN)-((R,R)-DepyPhos)-ruthenium(II)-tetrafluoroborat

In einem 50 ml Schlenkrohr mit Magnetrührer wird der nach Beispiel 1b) hergestellte Acetonitril-Komplex (η5-2,4-Dimethylpentadienyl)-(CH₃CN)₃ruthenium(II)-tetrafluoroborat in 15 ml Methylenchlorid gelöst und mit 25 mg (0,05 mmol) (R, R)-DepyPhos (Fa. Digital Speciality Chemicals, Toronto, Canada) bei Raumtemperatur für eine halbe Stunden gerührt. Zu der Lösung werden dann 50 ml n-Hexan getropft. Es fällt ein gelb-oranger Feststoff aus, der filtriert wird. Das Restlösemittel wird im Vakuum bei Raumtemperatur entfernt. Man erhält das Produkt in Form einer diastereomerenreinen Verbindung, Ausbeute 98%. Die P-P-Koordination des Liganden (und damit das Vorliegen eines 5-gliedrigen Ringes) wird durch das ³¹P NMR belegt.

### Verwendung zur katalytischen Hydrierung:

Der nach Beispiel 3 hergestellte Komplex wird zur asymmetrischen Hydrierung von *E*-2-Methyl-2-butensäure verwendet.Die Hydrierung erfolgt im Autoklaven unter 50 bar Wasserstoff, Lösungsmittel Methanol, Temperatur 50°C. Nach 20 Stunden Reaktionszeit wird der Wasserstoffdruck entfernt. Der Komplex liefert sehr gute Ergebnisse als Katalysator zur enantioselektiven katalytischen Hydrierung.

## Patentansprüche

1. Rutheniumkomplexe mit einem chiralen Di-Phosphor-Donorliganden für die homogene Katalyse gemäß der allgemeinen Formeln 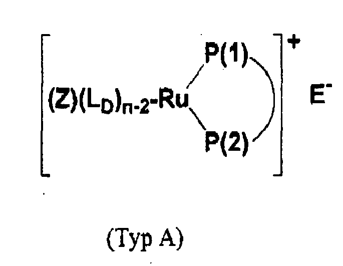 und 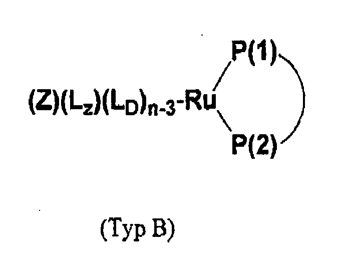 wobei
Ruthenium in der Oxydationsstufe +II vorliegt,
P(1)-P(2) ein chiraler Di-Phosphor-Donorligand,
n eine ganze Zahl mit n ≥ 3,
L_{D} mindestens ein neutraler Ligand,
Z ein π-gebundener anionischer offener Pentadienyl-Ligand
E⁻ ein Anion einer Sauerstoffsäure oder Komplexsäure und
L_{Z} mindestens ein anionischer Ligand ist,
und der chirale Di-Phosphor-Donorligand P(1)-P(2) eine bidentate P-P-
Koordination aufweist und mit dem Ruthenium einen vier- bis
sechsgliedrigen Ring bildet.

2. Rutheniumkomplexe nach Anspruch 1, wobei der chirale Di-Phosphor-Donorligand P(1)-P(2) ausgewählt ist aus der Gruppe der Diphosphine, Diphospholane, Diphosphite, Diphosphonite und Diazaphospholane.

3. Rutheniumkomplexe nach Anspruch 1 oder 2, wobei ein viergliedriger Ring vorliegt und der chirale Di-Phosphor-Donorligand P(1)-P(2) ausgewählt ist aus der Gruppe der MiniPhos und Trichickenfootphos Liganden.

4. Rutheniumkomplexe nach Anspruch 1 oder 2, wobei ein fünfgliedriger Ring vorliegt und der chirale Di-Phosphor-Donorligand P(1)-P(2) ausgewählt ist aus der Gruppe DIPAMP, Norphos, PROPHOS, DuPHOS, Chiraphos, Bis-P*-family, DepyPhos (oder DeguPhos), RoPhos, CATAXIUM, ButiPhane, (1,2-ethylen)-BinaPhane, (1,2-phenylen)-BinaPhane, Binapine, TangPhos, BPE, BasPhos, MalPhos, Me-KetalPhos, Helmchen-Liganden, PennPhos, UCAP, Hoge-Liganden sowie CNR-Phos Liganden.

5. Rutheniumkomplexe nach Anspruch 1 oder 2, wobei ein sechsgliedriger Ring vorliegt und der chirale Di-Phosphor-Donorligand P(1)-P(2) ausgewählt ist aus der Gruppe der BDPP (oder SKEWPHOS), BPPFOH, MandyPhos, JosiPhos, FerroTANE, Me-f-KetalPhos, Ferrocelane, Trifer sowie (1, 1 -ferrocene)-BinaPhane Liganden.

6. Rutheniumkomplexe nach einem der Ansprüche 1 bis 5, wobei L_{D} ein neutraler 2-Elektronen-Donorligand ist und ein Lösungsmittelligand aus der Gruppe Acetonitril (CH₃CN), Diethylether, Wasser, Aceton, Tetrahydrofuran, Dioxan, Pyridin, Imidazol oder Thiophen ist.

7. Rutheniumkomplexe nach einem der Ansprüche 1 bis 6, wobei Z ein substituierter offener Pentadienylligand ist und 1-Methylpentadienyl-, 2,4-Dimethyl-pentadienyl- sowie 2,3,4-Trimethylpentadienyl- umfasst.

8. Rutheniumkomplexe nach einem der Ansprüche 1 bis 7, wobei Z 2,4-Dimethylpentadienyl darstellt.

9. Rutheniumkomplexe nach einem der Ansprüche 1 bis 8, wobei E⁻ ein Anion aus der Gruppe HSO₄⁻, CF₃SO₃⁻, CF₃CO₂⁻ CH₃CO₂⁻, ClO₄⁻, BF₄⁻, B(Aryl)₄⁻, SbF₆⁻, oder PF₆⁻ ist.

10. Rutheniumkomplexe nach einem der Ansprüche 1 bis 9, wobei L_{Z} mindestens ein anionischer Ligand aus der Gruppe der Halogenide oder Pseudohalogenide ist.

11. Rutheniumkomplexe nach einem der Ansprüche 1 bis 10, wobei L_{z} Iodid, Z 2,4-Dimethylpentadienyl und E⁻ BF₄⁻ bedeutet.

12. Verfahren zur Herstellung des Rutheniumkomplexes (Typ A) nach einem der Ansprüche 1 bis 9, **dadurch gekennzeichnet, dass** eine Ru-Ausgangsverbindung der allgemeinen Formel
[Z-Ru-(L_{D})ₙ]⁺E⁻
bei der
Ru in der Oxydationsstufe +II vorliegt,
n eine ganze Zahl mit n ≥ 3,
L_{D} mindestens ein neutraler Ligand,
Z ein π-gebundener anionischer offener Pentadienyl-Ligand,
E⁻ ein Anion einer Sauerstoffsäure oder Komplexsäure ist,
mit einem chiralen Di-Phosphor-Donorliganden P(1)-P(2) umgesetzt wird.

13. Verfahren gemäß Anspruch 12, wobei der chirale Di-Phosphor-Donorligand P(1)-P(2) mit dem Ruthenium einen vier- bis sechsgliedrigen Ring bildet.

14. Verfahren nach Anspruch 12 oder 13, wobei der chirale Di-Phosphor-Donorligand P(1)-P(2) ausgewählt ist aus der Gruppe der Diphosphine, Diphospholane, Diphosphite, Diphosphonite und Diazaphospholane.

15. Verfahren nach einem der Ansprüche 12 bis 14, wobei der chirale Di-Phosphor-Donorligand P(1)-P(2) mit dem Ruthenium einen viergliedrigen Ring bildet und ausgewählt ist aus der Gruppe der MiniPhos und Trichickenfootphos Liganden.

16. Verfahren nach einem der Ansprüche 12 bis 14, wobei der chirale Di-Phosphor-Donorligand P(1)-P(2) mit dem Ruthenium einen fünfgliedrigen Ring bildet und ausgewählt ist aus der Gruppe der DIPAMP, Norphos, PROPHOS, DuPHOS, Chiraphos, Bis-P*-family, DepyPhos (oder DeguPhos), RoPhos, CATAXIUM, ButiPhane, (1,2-ethylen)-BinaPhane, (1,2-phenylen)-BinaPhane, Binapine, TangPhos, BPE, BasPhos, MalPhos, Me-KetalPhos, Helmchen-Liganden, PennPhos, UCAP, Hoge-Liganden und CNR-Phos Liganden.

17. Verfahren nach einem der Ansprüche 12 bis 14, wobei der chirale Di-Phosphor-Donorligand P(1)-P(2) mit dem Ruthenium einen sechsgliedrigen Ring bildet und ausgewählt ist aus der Gruppe BDPP (oder SKEWPHOS), BPPFOH, MandyPhos, JosiPhos, FerroTANE, Me-f-KetalPhos, Ferrocelane, Trifer sowie (1, 1 - ferrocene)-BinaPhane Liganden.

18. Verfahren nach einem der Ansprüche 12 bis 17, wobei L_{D} mindestens ein neutraler 2-Elektronen-Donorligand ist und ein Lösungsmittelligand aus der Gruppe Acetonitril (CH₃CN), Diethylether, Wasser, Aceton, Tetrahydrofuran, Dioxan, Pyridin, Imidazol oder Thiophen ist.

19. Verfahren nach einem der Ansprüche 12 bis 18, wobei Z ein substituierter offener Pentadienylligand ist und 1-Methylpentadienyl-, 2,4-Dimethylpentadienyl- sowie 2,3,4-Trimethylpentadienyl- umfasst.

20. Verfahren nach einem der Ansprüche 12 bis 19, wobei E⁻ ein Anion aus der Gruppe HSO₄⁻, CF₃SO₃⁻, CF₃CO₂⁻ CH₃CO₂⁻, ClO₄⁻, BF₄⁻, B(Aryl)₄⁻, SbF₆⁻ oder PF₆⁻ ist.

21. Verfahren nach einem der Ansprüche 12 bis 20, wobei als Lösungsmittel dipolare, aprotische Lösungsmittel wie Aceton, THF oder Dioxan verwendet werden.

22. Verfahren zur Herstellung des Rutheniumkomplexes (Typ B) nach einem der Ansprüche 1 bis 11, wobei der kationische Rutheniumkomplex des Typs A mit einem negativ geladenen Liganden L_{z} aus der Gruppe der Halogenide oder Pseudohalogenide umgesetzt wird.

23. Verfahren nach Anspruch 22, wobei als Lösungsmittel ein wässriges Lösungsmittelgemisch, insbesondere ein Gemisch eines dipolaren, aprotischen Lösungsmittels mit Wasser, verwendet wird.

24. Verwendung der Rutheniumkomplexe nach einem der Ansprüche 1 bis 11 als Katalysatoren für die homogene asymmetrische Katalyse, insbesondere für die homogene asymmetrische katalytische Hydrierung von organischen Verbindungen.

25. Verwendung der Rutheniumkomplexe nach einem der Ansprüche 1 bis 11 als Katalysatoren für die enantioselektiven Hydrierung von C=C-, C=O- oder C=N-Mehrfachbindungen.
